# EUROPÄISCHE PATENTANMELDUNG

(11) **EP 2 562 244 A2**
(43) Veröffentlichungstag der Anmeldung: **27.02.2013**
(21) Anmeldenummer: 12400026.6
(22) Anmeldetag: 10.07.2012
(51) Int. Cl.: C12M 1/107, B01F 7/00, B01F 7/02, B01F 7/32

(54) **Fermenter-Rührwerkseinheit**

(30) Priorität: 13.07.2011 DE 102011107234
(71) Anmelder: Spang, Harald, 54472 Monzelfeld (DE)
(72) Erfinder: Spang, Harald, 54472 Monzelfeld (DE)

(57) **Zusammenfassung**

Beschrieben wird eine Fermenter-Rührwerkeinheit für die Fermentation von Biomassen mit hohem Trockensubstanzgehalt nach dem Propfenstromverfahren auf Basis eines Rührwerkes (1), welches als Tragwerkkonstruktion, diese als gasdichte Rohrkonstruktion (Gitterrohrwelle) mit mehreren runden Abrollern (3) ausgeführt ist, der Antrieb der Rührwerkwelle über einen Ketten-, Zahnrad oder Riemenantrieb (6,7,8) erfolgt, die Rührwerkwelle frei in einer axialen und radialen Führung im Fermenter auf den Abrollerauflagern (4) aufliegt, die Abrollerauflager in einem auf dem Fermenterboden befestigten Rahmen (5) befestigt sind und die Abrollerauflager von außen über Flüssigkeit geschmiert werden.

## Beschreibung

Bei der Erfindung handelt es sich um eine Fermenter-Rührwerkeinheit für die Fermentation von Biomassen mit hohem Trockensubstanzgehalt nach dem Propfenstromverfahren auf Basis eines Rührwerkes, welches als Tragwerkkonstruktion ausgeführt ist.

Der Propfenstromfermenter ist in der Fermentation bekannt und hat sich zur Vergärung von Substraten wie Bioabfall, Grünschnitt aus der Landschaftspflege und weiteren trockenen Biomassen bereits bewährt. Die Fermentierung kann mesophil bei 38°C bis 42°C oder thermophil bei 50°C bis 55°C erfolgen.

Solche Fermenter-Rührwerkeinheiten sind bereits in CH686085A, DE102005057979A1, DE102005041798A1, DE19648875B4 und DE102007014417A1 beschrieben. Ein Propfenstromfermenter kann kontinuierlich beschickt werden. Die Raumbelastung, das heißt die Masse an organischer Biomasse pro Kubikmeter Fermentervolumen pro Tag liegt bei 6 kg bis 10 kg, wodurch hohe Energieumsätze bei kleinen Fermentervolumen erreicht werden.

Der Biomassepropfen bewegt sich dabei über eine Verweilzeit von 25 bis 35 Tagen durch den Fermenter und wird hierbei durch ein sich drehendes, in der Regel längs liegendes Rührwerk entgast, zum Teil transportiert und die Temperatur wird dabei gleichmäßig im Gärgut verteilt.

Da die liegenden Rührwerke eine Länge von 15 bis 25 m aufweisen, müssen diese hohe Drehmomente übertragen können. Die bisher konstruierten Propfenstromfermenter und Rührwerkwellen, beispielsweise beschrieben in CH686085A, sind sehr aufwendig konstruiert, statische und dynamische Lasten des Rührwerkes werden zum größten Teil in der Betonwand des Fermenters abgetragen oder müssen außerhalb des Fermenters abgefangen werden.

Die Welle wird zumeist beidseitig durch die Fermenterwand geführt und ist oft innerhalb des Fermenters zwischengelagert. Die Wanddurchdringungen müssen aufwändig gegen den Gärraum abgedichtet werden. Die Antriebsmotoren und Getriebe müssen große Drehmomente übertragen und haben dadurch große Abmessungen und Leistungsaufnahmen. Die Torsionskräfte auf die Welle sind sehr groß, so dass bei vielen Ausführungen das Rührwerk mit seiner Dimensionierung die Schwachstelle im System darstellt, beispielsweise beschrieben in DE102007014417A1.

Eine Ausnahme bildet hierbei das Linde KCA-Verfahren aus DE102005057979A1, welches mehrere quer zum Propfenstrom angeordnete Rührwellen hat. Hierbei ist nicht die Dimensionierung der Rührwellen die Schwachstelle, sondern die Vielzahl der Rührwellen, welche viele Wanddurchdringungen, Antriebseinheiten und einen hohen Regelaufwand benötigen.

All dieses wirkt sich negativ auf die Sicherheit, auf die Verfügbarkeit der Gesamtanlage, auf die Wartungs- und Reparaturkosten sowie auf die Investitionskosten aus. Bei steigenden Biomassepreisen und kleineren dezentralen Anlagen lässt sich damit keine Wirtschaftlichkeit erreichen.

Aufgabe der Erfindung ist die Konstruktion eines standardisierten, optimierten Pröpfenstromfermenters und einer Rührwerkwelle, die trotz einfachem Aufbau sehr stabil ist, hohe Drehmomente übertragen kann, hohe Verfügbarkeiten garantiert, einen niedrigen Energieverbrauch hat und schnell errichtet werden kann.

Gelöst wird diese Aufgabe bei einem Fermenter, beispielsweise einem Propfenstromfermenter, mit einem Langachsrührwerk für die Eigendurchmischung einer auszugasenden Biomasse mit hohem Trockensubstanzanteil in einem Gärraum durch die Ausführungen, dass die Rührwerkwelle im Fermenter selbst gelagert ist und somit die auftretenden Kräfte in die Bodenplatte abgetragen werden.

Die Welle ist entweder zwischengelagert oder freitragend auf Gleit- oder Rollenlagern stirnseitig innerhalb des Fermenters gelagert. Die Welle ist als Leichtbaukonstruktion ausgeführt und wird über einen Zahnrad-, Ketten- oder Riemenantrieb angetrieben. Der Antrieb der Rührwerkwelle erfolgt im Allgemeinen in der Mitte der Rührwerkwelle. Er kann alternativ auch stirnseitig ausgeführt werden. Der Antrieb der Rührwerkwelle erfolgt innerhalb oder außerhalb des Fermenters.

Durch die innen gelagerte Rührwerkwelle ist es konstruktiv möglich, alle mechanischen Kräfte in die Bodenplatte zu übertragen. Dadurch werden schwierig abzudichtende Wanddurchführungen, konstruktiv aufwendige Dimensionierungen der stirnseitigen Wände und Schwächung der Fermenterhülle durch große Wanddurchführungen vermieden.

Die Rührwerkwelle ist als Tragwerkskonstruktion (Gitterrohrwelle) mit mindestens zwei runden Abrollern ausgeführt. Diese Konstruktion zeichnet sich durch eine sehr hohe Biege- und Torsionssteifigkeit aus.

Der Ketten-, Zahnrad- oder Riemenantrieb erfolgt an einem der Abroller. Die Abroller haben im Allgemeinen einen Durchmesser im Bereich von drei bis vier Meter, wodurch das Drehmoment auf einem sehr großen Radius übertragen wird. Dadurch wird die notwendige Antriebskraft deutlich reduziert.

Die Rührwerkwelle liegt frei in einer axialen Führung auf den Abrollerauflagern. Sie kann ohne Entleerung des Fermenters herausgehoben werden. Sollte ein Rührwerkschaden auftreten, kann das Rührwerk in sehr kurzer Zeit repariert oder ersetzt werden, ohne dass der Fermentationsvorgang abgebrochen werden muss. Dieses garantiert unter anderem eine hohe Verfügbarkeit.

Die Rührwerkwelle ist als gasdichte Rohrkonstruktion ausgeführt. Dadurch erfährt die Welle im Gärsubstrat einen Auftrieb in Höhe von ca. 80 % ihres Eigengewichtes, wodurch die Auflager der Abroller sowie die Antriebseinheit entlastet werden.

Die Rührwerkwelle wird innerhalb des Fermenters angetrieben. Zur Reduzierung der Antriebsleistung wird eine Übersetzung über Zahnrad-, Ketten- oder Seitantriebe verwendet. Dadurch wird ein aufwendiges und teueres Übersetzungsgetriebe, wie beispielsweise ein Planetengetriebe, vermieden. Somit kann ein handelsüblicher Antriebsmotor mit einer kleinen Leistung eingesetzt werden.

Der Antriebsmotor der Welle wird innerhalb oder außerhalb des Fermenters angebracht. Die Drehmomentkräfte werden in die Bodenplatte eingeleitet.

Die Lager der Abroller werden von außen über Flüssigkeit geschmiert, wodurch keine Störstoffe wie beispielsweise Sand in die Lager eindringen können. Die Lager sind wartungsfrei.

Die Rührwerkwelle liegt in einem auf dem Fermenterboden befestigten Rahmen, in dem auch die Lager für die Abroller befestigt sind.

Da das Rührwerk nicht durch die Behälterwand geführt wird, ist die Behälterwand ohne erhöhte Statik als einfache Flüssigkeitsbehälterwand ausgeführt. Dadurch wird ebenfalls das Risiko einer möglichen Behälterhavarie in unmittelbarer Umgebung einer durch eine Vielzahl von eingelassenen Befestigungsbolzen und Schwerlastdübeln geschwächten Wellendurchführung vermieden, und die Investitionskosten werden reduziert.

Der Fermenter kann sowohl als Betonbauwerk in monolithischer- oder Fertigbauteilkonstruktion oder als Stahlbehälter ausgeführt werden.

Der Fermenter wird durch abnehmbare Platten aus Spannbeton, Kunststoff, Stahl oder durch ein Tragluftdach zur Atmosphäre hin verschlossen. Die Fermenterabdeckung kann schnell demontiert werden und das Rührwerk problemlos durch diese Öffnung bei voll gefülltem Fermenter ausgetauscht oder repariert werden.

Der Propfenstromfermenter ist je nach Anfall des geplanten Substrates als Einzelfermenter oder als Doppelfermenter ausgeführt.

Die Erfindung wird anhand der Abbildungen näher erläutert, in denen ein Ausführungsbeispiel schematisch dargestellt ist.

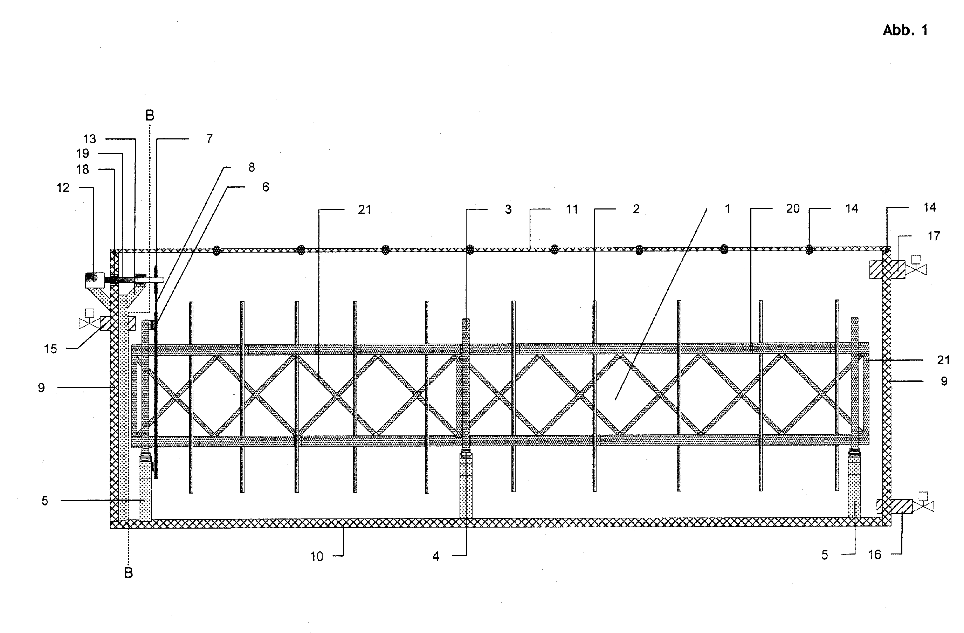

Abbildung 1 zeigt einen außermittigen Längsschnitt A-A durch einen Fermenter mit dem nach der Erfindung beschriebenen Langachsrührwerk.

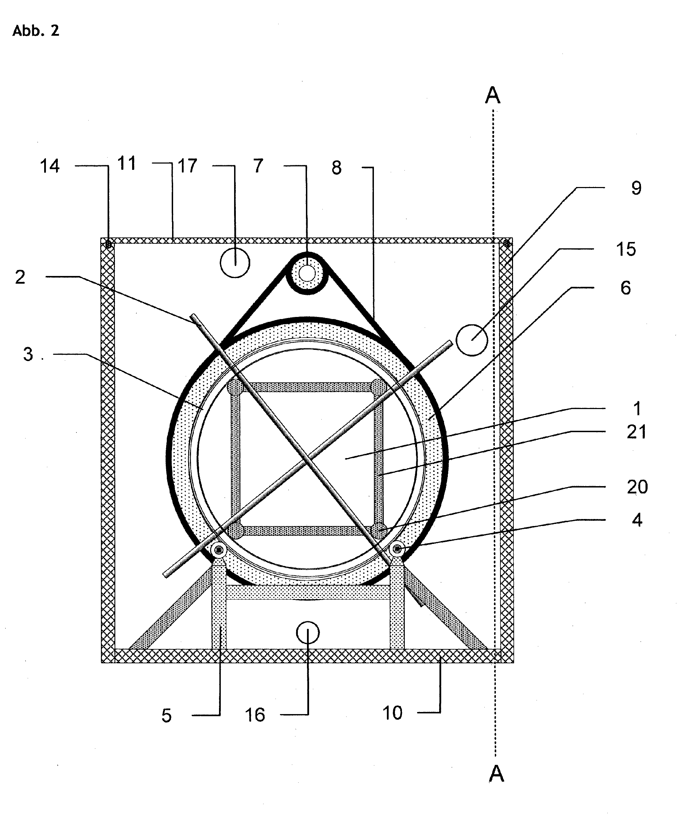

Abbildung 2 zeigt einen Querschnitt B-B durch einen Fermenter mit dem nach der Erfindung beschriebenen Langachsrührwerk.

Der in den Abbildungen dargestellte Fermenter ist als rechteckiger nach oben offener Baukörper ausgeführt. Dieser besitzt an den stirnseitigen Behälterwänden (9) Öffnungen. An der Eintragseite wird die zu vergärende Biomasse über eine Eintragsöffnung (15) eingebracht. An der gegenüberliegenden Stirnseite wird die vergorene Biomasse (Gärrest) über eine Austragsöffnung (16) entnommen. An der Eintragseite ist eine Durchdringung (18) für die Antriebseinheit der Rührwerkwelle ausgeführt. Weitere Öffnungen dienen der Gasentnahme (17) und der visuellen Überprüfung (Schaugläser). Alle Öffnungen sind gas- und flüssigkeitsdicht ausgeführt. Das Fermenterbauwerk, bestehend aus Boden (10) und Behälterwänden (9), ist oben mit abnehmbaren Spannbetonplatten (11) abgedeckt. Die Spannbetonplatten sind an den Berührungsflächen und zu den Behälterwänden hin jeweils mit Dichtungsmaterial (14) versehen und verschließen somit den Fermenter gasdicht.

Im Fermenter sind an den Stirnseiten und dazwischen jeweils eine Stahlkonstruktion (5) fest auf dem Fermenterboden (10) montiert. Die Stahlkonstruktion (5) dient der Aufnahme der Abrollerauflagern (4) und trägt die Rührwerkwelle (1).

An der Eintragseite ist eine Stahlkonstruktion (13) fest auf der Bodenplatte montiert. Die Stahlkonstruktion führt und trägt die Antriebswelle (19) des Antriebskettenrades (7). Der Antriebsmotor (12) treibt die Antriebswelle (19) an. Die Rührwerkwelle (1) wird aus vier quadratisch angeordneten Rohren (20), die untereinander durch Verstrebungen (21) fest miteinander verbunden sind, gebildet. Sie besteht aus gasdichten Rohren. An den Stellen der Abrollerauflagern (4) ist die Rührwerkwelle an runden Abrollern (3) befestigt. Die Abroller bestehen aus Doppel T-Trägerprofilen. An dem Abroller an der Einbringseite ist ein Zahnkranz (6) befestigt. Eine Kette (8) verbindet das Antriebszahnrad des Antriebsmotors (7) mit dem Zahnkranz (6) der Rührwerkwelle (1). Radial jeweils um 90° versetzt sind an der Rührwerkwerkwelle (1) Paddel (2) angebracht. Die Paddel bestehen aus gasdichten Rohren. Diese stehen diagonal zwischen den Rohren (20) der Rührwerkwelle (1).

### Bezugszeichenliste:

- 1.: Langachsrührwerk
- 2.: Radialpaddel
- 3.: Abroller
- 4.: Abrollerauflager
- 5.: Befestigung Abrollerauflager
- 6.: Antrieb Zahnrad Rührwerkwelle
- 7.: Antrieb Zahnrad Motor
- 8.: Antrieb (Kette, Riemen)
- 9.: Fermenterwand
- 10.: Fermenterboden
- 11.: Fermenterabdeckung
- 12.: Antriebsmotor
- 13.: Befestigung Antriebseinheit
- 14.: Abdichtung Fermenterabdeckung
- 15.: Eintragsöffnung
- 16.: Austragsöffnung
- 17.: Öffnung Gasentnahme
- 18.: Durchdringung Antriebswelle
- 19.: Antriebswelle
- 20.: Längsrohre Rührwerkwelle
- 21.: Verstrebungen Rührwerkwelle

## Patentansprüche

1. Fermenter-Rührwerkeinheit zum Durchmischen einer zu vergärenden Biomasse **dadurch gekennzeichnet, dass**
- das Rührwerk als Tragwerkskonstruktion mit mehreren runden Abrollern ausgeführt ist,
- diese Tragwerkskonstruktion als gasdichte Rohrkonstruktion ausgeführt ist,
- die Fermenter-Rührwerkeinheit eine Antriebseinheit enthält,
- der Antrieb für die Rührwerkwelle über einen Ketten-, Zahnrad oder Riemenantrieb erfolgt,
- der Antrieb der Rührwerkwelle mittig oder an den Stirnseiten der Rührwerkwelle eingebaut ist,
- die Rührwerkwelle frei in einer axialen und radialen Führung im Fermenter auf den Abrollerauflagetn aufliegt,
- die Abrollerauflager in einem auf dem Fermenterboden befestigten Rahmen befestigt sind, so dass die Drehmomente der Rührwerkwelle in die Bodenplatte eingeleitet werden

2. Fermenter-Rührwerkeinheit nach Anspruch 1 **dadurch gekennzeichnet, dass** der Antriebsmotor der Rührwerkwelle entweder innerhalb oder außerhalb des Fermenters angebracht ist.

3. Fermenter-Rührwerkeinheit nach einem der vorhergehenden Ansprüche **dadurch gekennzeichnet, dass** die Rührwerkwelle zur freien Entnahme im Fermenter eingebaut ist.

4. Fermenter-Rührwerkeinheit nach einem der vorhergehenden Ansprüche **dadurch gekennzeichnet, dass** der Fermenter nach oben hin offen ist und der Gasraum durch abnehmbare Platten beispielsweise aus Spannbeton, Kunststoff, Stahl oder durch ein Tragluftdach zur Atmosphäre hin verschlossen ist.

5. Fermenter-Rührwerkeinheit nach einem der vorhergehenden Ansprüche **dadurch gekennzeichnet, dass** diese als Einzelfermenter oder als Doppelfermenter ausgeführt ist.

6. Fermenter-Rührwerkeinheit nach einem der vorhergehenden Ansprüche **dadurch gekennzeichnet, dass** über die Länge des Rührwerkes verteilt mehrere radial angeordnete gasdichte Paddel fixiert sind.

7. Fermenter-Rührwerkeinheit nach einem der vorhergehenden Ansprüche **dadurch gekennzeichnet, dass** die Abrollerauflager von außen über Flüssigkeit geschmiert und die Lager wartungsfrei sind.
